# EUROPEAN PATENT APPLICATION

(11) **EP 4 119 661 A1**
(43) Date of publication of application: **18.01.2023**
(21) Application number: 21217370.2
(22) Date of filing: 23.12.2021
(51) Int. Cl.: C12N 9/16, A23K 10/16

(54) **PHYTASE VARIANTS WITH IMPROVED STABILITY AND IP4 ACTIVITY**

(30) Priority: 16.07.2021 EP 21186223; 16.07.2021 EP 21186231
(71) Applicant: AB Enzymes Oy, 05200 Rajamäki (FI)
(72) Inventor: JUNTUNEN, Kari, 05200 Rajamäki (FI); AHOLA, Pihla, 05200 Rajamäki (FI); JOKINEN, Laura, 05200 Rajamäki (FI); METZGER, Tara, 64293 Darmstadt (DE); KÜHN, Imke, 64293 Darmstadt (DE); PURANEN, Terhi, 05200 Rajamäki (FI); PALOHEIMO, Marja, 05200 Rajamäki (FI)
(74) Representative: Espatent Oy

(57) **Abstract**

A variant polypeptide of phytase enzyme is disclosed having increased IP4 activity and improved thermostability, as well as compositions containing said variant polypeptide, recombinant host cells for producing it, methods for its production, uses and methods wherein said variant polypeptide is used, and feed products containing said variant polypeptide.

## Description

### TECHNICAL FIELD

The present disclosure generally relates to field of enzyme technology. The disclosure relates particularly, though not exclusively, to phytase variants having improved thermostability and IP4 degradation activity. The present phytase variants are useful in various applications where degradation of phytate is desired in demanding environmental, production, and processing conditions, such as in feedstuffs.

### BACKGROUND

This section illustrates useful background information without admission of any technique described herein representative of the state of the art.

Phytic acid (phytate, inositol hexakisphosphate, IP6) is found in many plants where it functions as storage of phosphate. Phosphate stored in IP6 molecules can be released as inorganic phosphate. When inorganic acid is released from myo-inositol hexakisphosphate (IP6), it is converted at first to myo-inositol pentakisphosphate (IP5), and then further via myo-inositol tetrakisphosphate (IP4), myo-inositol trisphosphate (IP3), myo-inositol bisphosphate (IP2) to myo-inositol monophosphate (IP1).

Phytases are a group of phosphatase enzymes that catalyse the hydrolysis of phytic acid. Commercially available phytases belong to the histidine acid phosphatase (HAP) protein family. The phytases belonging to the HAP protein family share a conserved N-terminal active site hepta-peptide motif RHGXRXP and a catalytically active HD-dipeptide at the C-terminus. Histidine acid phosphatases are part of a larger superfamily of histidine phosphatases. They share a conserved catalytic core centred on a histidine, which becomes phosphorylated during the reaction. PFAM motif His_Phos_2 (PF00328) represents branch 2 of the histidine phosphatase superfamily, the branch containing mainly acid phosphatases and phytases.

Phytase enzymes can be used in feeds to improve phosphate availability from feed ingredients of plant origin (e.g., wheat, barley, corn, soybean) by phytate degradation. This is in particular of interest for monogastric animals like poultry, fish and pigs, because intestinal phytate degradation in the upper intestinal tract is limited in these animals. This limitation not only restricts utilisation of phosphorus, but also the availability of other nutrients due to the chelating effect of inositol phosphates (IPs), particularly by the high IPs, that is IP6, IP5, and IP4. Consequently, at least IP6 to IP4 should be dephosphorylated as completely as possible, but the dephosphorylation should preferably continue even to IP1 to further increase availability of phosphate and other nutrients.

Phytate breakdown by phytases is associated with a stepwise degradation of IP6 to lower inositol phosphate esters (IP5, IP4, IP3, IP2, and IP1). The use of industry standard levels of phytase have, expectedly, shown to significantly reduce IP6 levels *in vitro* and *in vivo.* However, in IP6 degradation experiments an increase of IP4 and IP3 has been detected in ileal digesta which shows that the hydrolytic cleavage of the first phosphate group is not the only limiting step in phytate degradation (Zeller et al, 2015). As even these lower inositol phosphates have antinutritive properties due to binding of different nutrients like minerals to them, the target in animal feeding is to degrade IP esters up to the terminal ileum (Bedford and Walk, 2016). Another reason for the aim to get inositol fully released is that increasing the content of free inositol has been shown to improve growth performance in animals by different mechanisms, which is still under investigation.

The part in the intestinal tract of animals where optimal degradation of inositol phosphate esters takes place with phytase supplements is the stomach due to its low pH, leading to the best substrate (phytate) solubility. Retention time in the stomach is short and part of the content might flush rapidly to the intestinal tract in which the pH is neutral. Therefore, further development of phytases acting quickly and more effectively on IP6 and, also on lower inositol phosphates like IP4 and IP3 isomers, is of relevance for the animal feeding industry. These type of phytases would further improve intestinal availability of phosphate and inositol.

It is an object of the present disclosure to provide phytase variants that exhibit phytase activity and that have improved properties that allow their use in industrial processes. Yet another object of the present disclosure is to provide phytase variants that can be used in enzyme compositions and feeds for phytate degradation.

### SUMMARY

The appended claims define the scope of protection. Any example and/or technical description of an apparatus, system, product and/or process in the description and/or drawing which is not covered by the claims is presented herein not as an embodiment of the invention but as an example useful for understanding the invention.

According to a first aspect is provided a variant polypeptide comprising an amino acid sequence having at least 80% amino acid sequence identity with SEQ ID NO: 1 and comprising amino acid substitutions at:
the position 126; and
at least one position selected from 30, 80, 94, 118, 176, 179, 212, 224, 227, 253, 287, 315, and 380; and
wherein the variant polypeptide has phytase activity; and
wherein the amino acid numbering corresponds to the amino acid numbering of the SEQ ID NO: 1.

In an embodiment the variant polypeptide has a histidine at the position 126.

In an embodiment the variant further comprises a substitution at the position 121 and/or 216.

In an embodiment the substitution at the position 121 and/or 216 is selected from 121K and 216T, preferably selected from P121K and M216T.

In an embodiment the variant polypeptide has an increased IP4 degradation activity compared to a phytase having the amino acid sequence SEQ ID NO: 1. In some embodiments the increased IP4 degradation activity means that the ratio of IP4 degradation activity to IP6 degradation activity is increased.

In an embodiment the variant polypeptide comprises at least one further amino acid substitution at a position selected from 56, 67, 107, 154, 174, 180, 183, 202, 204, 211, 258, 271, 276, 277, 285, 302, 344, 352, and 395.

In an embodiment the at least one further amino acid substitution results into the presence of at least one of the following amino acids: 56S, 67I, 107N, 154N, 174E, 180N, 183A, 202S, 204N, 204T, 211V, 258N, 271I, 276M, 277A, 285E, 302A, 344D, 352M, or 395A.

In an embodiment the at least one further amino acid substitution is a substitution selected from A56S, V67I, D107N, D154N, Q174E, K180N, K183A, A202S, D204N, D204T, W211V, Q258N, L271I, K276M, T277A, Q285E, G302A, N344D, L352M, or G395A.

In an embodiment the variant polypeptide has an unfolding temperature of at least 88°C, preferably at least 89°C, and even more preferably at least 90°C. The unfolding temperature can be determined as described in Example 4.

In an embodiment the variant polypeptide has an improved thermostability compared to a phytase having the amino acid sequence SEQ ID NO: 1. Thermostability can be determined for example as described in Example 5.

In a further embodiment improved thermostability means that the variant polypeptide has a higher melting temperature (Tₘ) than the parent enzyme, i.e. a phytase having the amino acid sequence SEQ ID NO: 1.

In an embodiment the variant polypeptide comprises:
a set of substitutions at the positions 30, 80, 94, 118, 121, 126, 176, 179, 180, 183, 204, 211, 212, 216, 224, 227, 253, 276, 277, 287, 315, 352, and 380; or a set of substitutions 30R, 80P, 94L, 118L, 121K, 126H, 176P, 179K, 180N, 183A, 204N, 211V, 212G, 216T, 224E, 227E, 253Y, 276M, 277A, 287S, 315G, 352M, and 380P; or
a set of substitutions Q30R, S80P, R94L, T118L, P121K, N126H, N176P, L179K, K180N, K183A, D204N, W211V, S212G, M216T, Q224E, Q227E, V253Y, K276M, T277A, Q287S, E315G, L352M, and A380P.

In an embodiment the variant polypeptide comprises:
a set of substitutions at the positions 30, 80, 94, 118, 126, 176, 179, 180, 183, 204, 211, 212, 216, 224, 227, 253, 276, 277, 287, 315, 352, and 380; or
a set of substitutions 30R, 80P, 94L, 118L, 126H, 176P, 179K, 180N, 183A, 204N, 211V, 212G, 216T, 224E, 227E, 253Y, 276M, 277A, 287S, 315G, 352M,
and 380P; or
a set of substitutions Q30R, S80P, R94L, T118L, N126H, N176P, L179K, K180N, K183A, D204N, W211V, S212G, M216T, Q224E, Q227E, V253Y, K276M, T277A, Q287S, E315G, L352M, and A380P.

In an embodiment the variant polypeptide comprises:
a set of substitutions at the positions 30, 56, 67, 80, 94, 107, 118, 121, 126, 154, 174, 176, 179, 180, 202, 204, 211, 212, 224, 227, 253, 271, 276, 277, 285, 287, 302, 315, 344, 352, 380, and 395; or
a set of substitutions 30R, 56S, 671, 80P, 94L, 107N, 118L, 121K, 126H, 154N, 174E, 176P, 179K, 180N, 202S, 204N, 211V, 212G, 224E, 227E, 253Y, 271I, 276M, 277A, 285E, 287S, 302A, 315G, 344D, 352M, 380P, and 395A; or
a set of substitutions Q30R, A56S, V67I, S80P, R94L, D107N, T118L, P121K, N126H, D154N, Q174E, N176P, L179K, K180N, A202S, D204N, W211V, S212G, Q224E, Q227E, V253Y, L271I, K276M, T277A, Q285E, Q287S, G302A, E315G, N344D, L352M, A380P, and G395A.

In an embodiment the variant polypeptide comprises:
a set of substitutions at positions 30, 56, 67, 80, 94, 107, 118, 126, 154, 174, 176, 179, 180, 202, 204, 211, 212, 216, 224, 227, 253, 271, 276, 277, 285, 287, 302, 315, 344, 352, 380, and 395; or
a set of substitutions 30R, 56S, 67I, 80P, 94L, 107N, 118L, 126H, 154N, 174E, 176P, 179K, 180N, 202S, 204N, 211V, 212G, 216T, 224E, 227E, 253Y, 2711, 276M, 277A, 285E, 287S, 302A, 315G, 344D, 352M, 380P, and 395A; or a set of substitutions Q30R, A56S, V67I, S80P, R94L, D107N, T118L, N126H, D154N, Q174E, N176P, L179K, K180N, A202S, D204N, W211V, S212G, M216T, Q224E, Q227E, V253Y, L271I, K276M, T277A, Q285E, Q287S, G302A, E315G, N344D, L352M, A380P, and G395A.

According to a second aspect is provided a recombinant host cell comprising genetic elements that allow producing at least one of the present variant polypeptides, and wherein the host cell is preferably selected from the group consisting of
a. filamentous fungal cells from Division *Ascomycota,* Subdivision *Pezizomycotina;* preferably from the group consisting of members of the Class *Sordariomycetes* or *Eurotiomycetes,* Subclass *Hypocreomycetidae* or *Sordariomycetidae* or *Eurotiomycetidae,* Orders *Hypocreales* or *Sordariales* or *Eurotiales,* Families *Hypocreacea* or *Nectriacea* or *Chaetomiaceae* or *Aspergillaceae,* Genera *Trichoderma* (anamorph of *Hypocrea)* or *Fusarium* or *Acremonium* or *Humicola* or *Thermothelomyces* or *Myceliophthora* or *Aspergillus;*
   more preferably from the group consisting of species *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Acremonium (Cephalosporium) chrysogenum, Humicola insolens, H. grisea, Thermothelomyces thermophilus, Myceliophthora thermophila, Aspergillus niger, A. niger* var. *awamori* and *A. oryzae;*
b. bacterial cells, preferably gram-positive *Bacilli* such as *B*. *subtilis, B*. *licheniformis, B*. *megaterium, B*. *amyloliquefaciens, B*. *pumilus,* gram negative bacteria such as *Escherichia coli,* actinomycetales such as *Streptomyces* sp.; and
c. yeasts, such as *Saccharomyces cerevisiae, Schizosaccharomyces pombe, Pichia pastoris, Yarrowia lipolytica;* and
   more preferably the host cell is selected from filamentous fungal cells such as *Trichoderma,* or from gram-positive Bacilli such as *Bacillus;*
most preferably from *Trichoderma reesei* or from *Bacillus subtilis* or *B*. *pumilus* or *B*. *licheniformis* or *B*. *amyloliquefaciens.*

In an embodiment the recombinant host cell comprises genetic elements configured to produce at least one of the present variant polypeptides, and wherein the host cell is a transgenic plant cell.

According to a third aspect is provided an enzyme composition comprising at least one of the present variant polypeptides, and optionally a preservative, and/or a carrier.

According to a fourth aspect is provided a use, and a method of using, of at least one of the present variant polypeptides or the present enzyme composition in the manufacturing of feedstuff or foodstuff, feed additive, a dietary supplement, or a pharmaceutical, comprising mixing the variant polypeptide or the enzyme composition with the feedstuff, foodstuff, feed additive, dietary supplement, or pharmaceutical. The use or the method of using preferably comprises at least adding the present variant polypeptide or enzyme composition and mixing.

According to a fifth aspect is provided a method of manufacturing the present variant polypeptide comprising:
a. providing a polynucleotide comprising genetic elements arranged to produce the present variant polypeptide; and
b. expressing the polynucleotide in a recombinant host cell, preferably in the present recombinant host cell.

According to a sixth aspect is provided an animal feed comprising the present variant polypeptide or the present enzyme composition, and at least one protein source of plant origin, and
a. optionally at least one further enzyme selected from protease, amylase, phytase, xylanase, endoglucanase, beta-glucanase, mannanase, cellulase, or a combination thereof; and
b. optionally at least one carrier or ingredient selected from maltodextrin, flour, salt, sodium chloride, sulphate, sodium sulphate, or a combination thereof.
According to a seventh aspect is provided a feed supplement comprising the present variant polypeptide or the present enzyme composition; and
a. optionally at least one further enzyme selected from protease, amylase, phytase, xylanase, endoglucanase, beta-glucanase, mannanase, cellulase, or a combination thereof; and
b. optionally at least one carrier or ingredient selected from maltodextrin, flour, salt, sodium chloride, sulphate, sodium sulphate, minerals, amino acids, prebiotics, probiotics, vitamins, or a combination thereof.

In an embodiment the feed supplement is a feed premix. The feed premix can be added to the feed before use.

According to an eighth aspect is provided a method of degrading or modifying material containing phytic acid or phytate, comprising treating said material with an effective amount of the present variant polypeptide or the present enzyme composition.

In an embodiment the treating comprises adding an effective amount of the variant polypeptide to the material preferably in an aqueous medium.

### BRIEF DESCRIPTION OF THE FIGURES

Some example embodiments will be described with reference to the accompanying figures, in which:

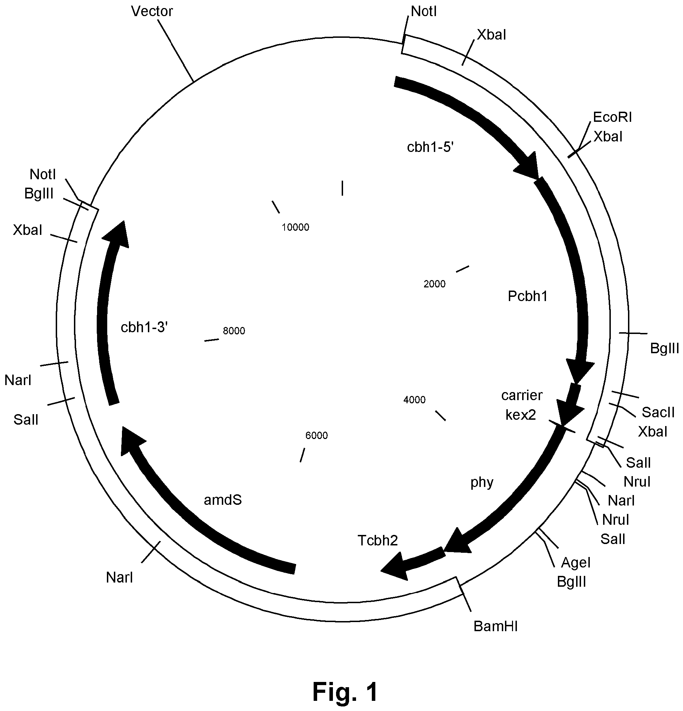

**Figure 1** shows a schematic picture of the expression plasmid used in the transformation of *Trichoderma reesei* for expression of parent phytase (SEQ ID NO:1) and phytase variant genes (phy). The expression of the recombinant genes in the host cell was controlled by use of the following genetic elements: *T. reesei cbh1* promoter (Pcbh1) for transcription initiation, and *T. reesei cbh2* (Tcbh2) terminator for transcription termination. *T. reesei cbh2* carrier encoding the CBHII CBM and linker region was used instead of the native phytase signal sequence and Kex2 protease cleavage site *(kex2)* was included between the encoded carrier polypeptide and phytase. A synthetic gene *(amdS)* encoding the AmdS marker was included for selection of the transformants and *T. reesei cbh1* 3'- and 5'-flanking regions (cbh1-3' and cbh1-5', respectively) were used to optionally target the expression cassette to *cbh1* locus. The vector part (Vector) derives from pUC19. Picture was generated using Clone Manager Professional 9 from Sci-Ed Software. A selection of restriction enzyme sites is shown in the picture.

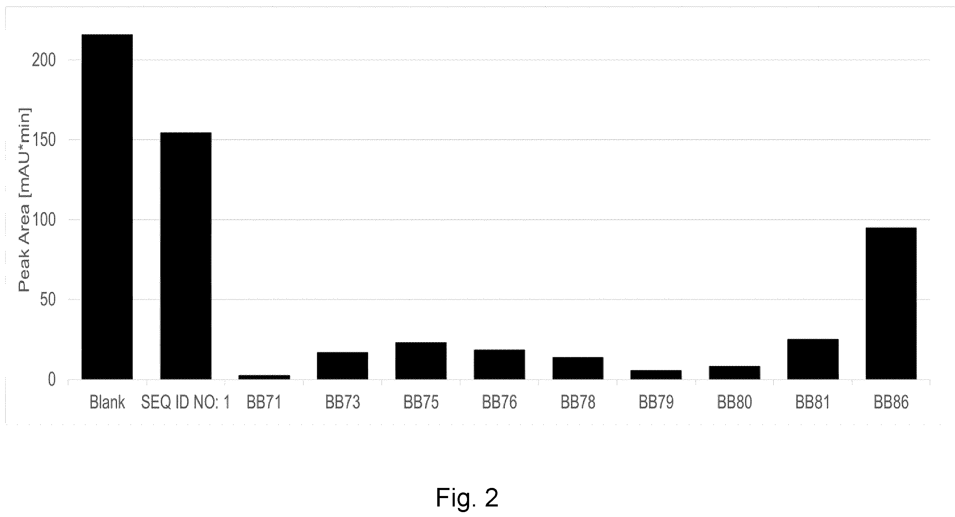

**Figure 2** shows the in vitro test (GIT) outcome for selected phytase variants that are improved in inositol phosphate degradation. The residual amount of the sum of IP6+IP5+IP4 after treatment of a corn-soybean meal based broiler feed with the tested phytases is shown. The dosing of the phytases was 500 FTU per kg feed and values are given as the sum of the IP6+IP5+IP4 peak areas analysed by HPLC.

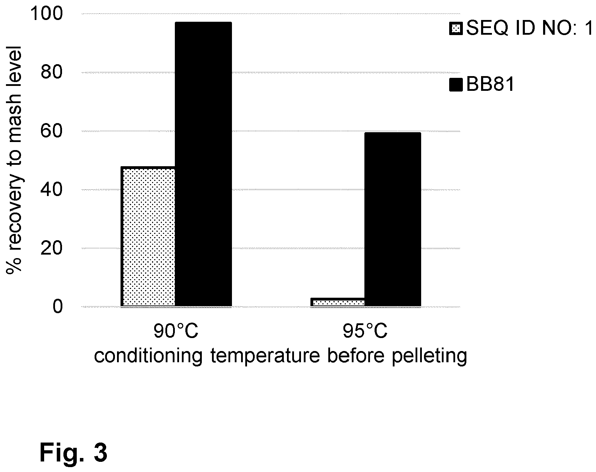

**Figure 3** shows recovery of phytases SEQ ID NO:1 and variant BB81 after 90°C and 95°C conditioning followed by feed pelleting. The recovery referred to the activity analysed in the untreated mash feed.

### SEQUENCE LISTING

- SEQ ID NO: 1: Amino acid sequence of parent phytase without signal peptide, also referred to herein with the name QPT2.
- SEQ ID NO: 2: Amino acid sequence of parent phytase variant without signal peptide comprising the following amino acid substitutions compared to SEQ ID NO: 1: Q30R, S80P, R94L, T118L, P121K, N126H, N176P, L179K, K180N, K183A, D204N, W211V, S212G, M216T, Q224E, Q227E, V253Y, K276M, T277A, Q287S, E315G, L352M, A380P, also referred to herein with the name BB71.
- SEQ ID NO: 3: Amino acid sequence of parent phytase variant without signal peptide comprising the following amino acid substitutions compared to SEQ ID NO: 1: Q30R, S80P, R94L, T118L, N126H, N176P, L179K, K180N, K183A, D204N, W211V, S212G, M216T, Q224E, Q227E, V253Y, K276M, T277A, Q287S, E315G, L352M, A380P, also referred to herein with the name BB73.
- SEQ ID NO: 4: Amino acid sequence of parent phytase variant without signal peptide comprising the following amino acid substitutions compared to SEQ ID NO: 1: Q30R, A56S, V67I, S80P, R94L, D107N, T118L, P121K, N126H, D154N, Q174E, N176P, L179K, K180N, A202S, D204N, W211V, S212G, Q224E, Q227E, V253Y, L271I, K276M, T277A, Q285E, Q287S, G302A, E315G, N344D, L352M, A380P, G395A, also referred to herein with the name BB78.
- SEQ ID NO: 5: Amino acid sequence of parent phytase variant without signal peptide comprising the following amino acid substitutions compared to SEQ ID NO: 1: Q30R, A56S, V67I, S80P, R94L, D107N, T118L, N126H, D154N, Q174E, N176P, L179K, K180N, A202S, D204N, W211V, S212G, M216T, Q224E, Q227E, V253Y, L271I, K276M, T277A, Q285E, Q287S, G302A, E315G, N344D, L352M, A380P, G395A, also referred to herein with the name BB81.

### DETAILED DESCRIPTION

As used herein, the term "phytase" means an enzyme having a capability to enzymatically degrade phytic acid to lower inositol phosphates.

Phytases are classified into 3-, 5- or 6-phytases (EC 3.1.3.8, EC 3.1.3.72, and EC 3.1.3.26, respectively) based on the carbon position on the inositol ring at which they preferably initiate phosphate hydrolysis. 6-phytases preferably first remove the phosphate group at the C6 position.

In one embodiment is provided a functional fragment of the variant polypeptide. A functional fragment is a shorter polypeptide of the variant polypeptide which does not contain all amino acids of the variant polypeptide, but which still is functionally the same or similar as the variant polypeptide. Functional similarity can be for example similar thermostability and/or similar enzymatic activity.

The present invention also relates to a polynucleotide comprising a nucleic acid sequence encoding a phytase variant polypeptide according to the first aspect.

In an embodiment the polypeptide comprising the phytase variant comprises at least one further amino acid sequence selected from a signal sequence, a secretory sequence, a carrier polypeptide, binding domain, a tag, a linker, an enzyme, or any combination thereof.

The terms "phytase variant", "variant of phytase", or "variant polypeptide" mean a phytase molecule obtained by site-directed or random mutagenesis, insertion, substitution, deletion, recombination and/or any other protein engineering method, and which leads into a genetically modified phytase that differs in its amino acid sequence from the parent phytase such as a wild type phytase. The terms "wild type phytase ", "wild type enzyme", "wild type", or "wt" in accordance with the disclosure, refer to a phytase enzyme with an amino acid sequence found in nature, or a fragment thereof. The variant encoding gene can be synthesised or the parent gene be modified using genetic methods, e.g., by site-directed mutagenesis, a technique in which one or more than one mutation are introduced at one or more defined sites in a polynucleotide encoding the parent polypeptide. The term variant phytase may also be referred to by using a name given to the variant in examples, tables, figures, or claims.

The present variants are preferably recombinantly produced non-naturally occurring proteins. They can be prepared using generally known recombinant DNA technology. Briefly, the polynucleotide encoding a variant is cloned and inserted into an expression vector, transformed into a host cell, and then expressed. Preferably, mutations are introduced into the coding sequence with codons preferred by the selected host strain. Methods for protein production by recombinant technology in different host systems are known in the art. Preferably, the variants are produced as extracellular proteins that are secreted into the culture medium wherein the host cell is cultured, and from which they can be easily recovered and isolated.

As used herein, the term "mature polypeptide" means any polypeptide wherein at least one signal sequence or signal peptide or signal peptide and a putative pro-peptide, or a carrier peptide or a fusion partner is cleaved off. As used herein, a "peptide" and a "polypeptide" are amino acid sequences including a plurality of consecutive polymerized amino acid residues. For purpose of this disclosure, peptides are molecules including up to 20 amino acid residues, and polypeptides include more than 20 amino acid residues.

The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

As used herein, "sequence identity" means the percentage of exact matches of amino acid residues between two optimally aligned sequences over the number of positions where there are residues present in both sequences. When one sequence has a residue with no corresponding residue in the other sequence, the alignment program allows a gap in the alignment, and that position is not counted in the denominator of the sequence identity calculation.

In one embodiment "sequence alignment" of the amino acid sequences means aligning the sequences using Clustal Omega (1.2.4) multiple sequence alignment program (https://www.ebi.ac.uk/Tools/msa/clustalo/) as described by Sievers et al. 2011, and using the default settings.

Unless otherwise specified, all references to a certain amino acid position refer to an amino acid of the SEQ ID NO: 1 in said position, or to an amino acid present or missing in the corresponding position of an amino acid sequence aligned with SEQ ID NO: 1.

As used herein, the term "disulfide bridge", or "disulfide bond", or "SS bridge" refers to a bond formed between the sulfur atoms of cysteine residues in a polypeptide or a protein. Disulfide bridges can be naturally occurring, or non-naturally occurring, and, for example, introduced by way of amino acid substitution(s).

As used herein, the term "corresponding positions" or "corresponding amino acid position" means aligning at least two amino acid sequences according to identified regions of similarity or identity as pairwise alignment or as multiple sequence alignment, thereby pairing up the corresponding amino acids. In the present disclosure corresponding positions typically refer to a position corresponding to the position in SEQ ID NO: 1.

As used herein, "amino acid substitution" means an amino acid residue replacement with an amino acid residue that is different than the original amino acid in that specific replacement position. The term "amino acid substitution" can refer to conservative amino acid substitutions or to non-conservative amino acid substitutions, which means the amino acid residue is replaced with an amino acid residue having a similar side chain (conservative), or a different side chain (non-conservative), as the original amino acid residue in that place.

The term "secretory signal sequence" or "signal sequence" or a "secretory peptide" refers to an amino acid sequence which is a component or a part of a larger polypeptide, and which directs the larger polypeptide through a secretory pathway of a host cell in which it is produced. The secretory signal sequence can be native to the polypeptide or it can be obtained from another source. Depending on the host cell, the larger polypeptide may be cleaved off from the secretory peptide during transit through the secretory pathway, thereby forming a mature polypeptide lacking the secretory peptide.

The term carrier polypeptide or fusion partner refers to a polypeptide into which the protein of interest (phytase) is translationally fused for example to improve yield. The carrier/fusion partner can be either homologous or heterologous to production host in its origin and can be a full-length protein or a fragment of a protein (e.g., a core, a CBM or a CBM and linker region). It is preferably encoded by a gene or a nucleotide sequence with good expression level.

"Phytase activity" as used herein, refers to the phytic acid degrading activity. Example 3 provides a method for determining phytase activity. Accordingly, IP4 activity refers to the capability to degrade IP4, and IP6 activity refers to the capability to degrade IP6. The ratio of IP4 degrading activity to IP6 degrading activity is sometimes disclosed herein in as IP4/IP6 activity.

In an embodiment the term "enzyme composition" means an enzymatic fermentation product, possibly isolated and purified, typically produced by a pure culture of a microorganism. The enzyme composition usually comprises a number of different enzymatic activities produced by the microorganism. In another embodiment the enzyme composition is a mixture of monocomponent enzymes, preferably enzymes derived from bacterial or fungal species by using conventional recombinant production techniques. The enzyme composition may contain for example stabilators and/or preservatives which prevent microbial growth and improve storage stability. A filler component such as maltodextrin, flour, and/or salt may also included in an enzyme composition.

As used herein, a "host cell" means any cell type that is susceptible to transformation, transfection, transduction, mating, crossing, CRISPR-Cas, or the like with a nucleic acid construct or expression vector comprising a polynucleotide. The term "host cell" encompasses any progeny that is not identical due to mutations that occur during replication. Non-limiting examples of a host cell are fungal cells, preferably a filamentous fungal cell (e.g., *Trichoderma* or *Trichoderma reesei, Aspergillus* or *Aspergillus oryzae* or *A. niger, Thermothelomyces* or *Thermothelomyces heterothallica, Myceliophthora* or *Myceliophthora thermophila,* or *Humicola* or *Humicola insolens* or *Fusarium* or *Fusarium venenatum),* bacterial cells, preferably gram-positive *Bacilli* (e.g., *Bacillus subtilis, B*. *licheniformis, B*. *megaterium, B*. *amyloliquefaciens, B*. *pumilus),* gram-negative bacteria (e.g., *Escherichia coli), actinomycetales* (e.g., *Streptomyces sp., Nonomuraea flexuosa)* and yeasts (e.g., *Saccharomyces cerevisiae, Schizosaccharomyces pombe, Pichia pastoris, Yarrowia lipolytica).*

In another embodiment the phytase variant is obtained by recombinant production in plant cells, i.e. in a transgenic plant.

The recombinant host cell can be used to produce the phytase variant and to contain a polynucleotide encoding it. The recombinant host cell can be operably linked to one or more control sequence to direct the production of the variant, and that make it possible to initiate the production of the present phytase variant by a stimulus, as is known in the field. The recombinant host cell is useful also in preparation of variants with different properties. For example, a host cell can be selected, which provides post-translational modifications beneficial for stability or activity, or which facilitates post-processing of a variant produced in the host cell.

In an embodiment the host cell is non-pathogenic. This is particularly advantageous for using the host cell, or the phytase variant produced in it, for animal feed products.

In an embodiment the composition containing the phytase variant is food or feed, and it may further comprise plant material which contains phytic acid.

In an embodiment the composition is a food additive or a feed additive further comprising at least one of: at least one trace mineral, at least one amino acid, in particular lysine, water soluble vitamin, fat soluble vitamin, prebiotic, and probiotic.

In an embodiment the composition is a food additive or a feed additive complying with the requirements of Regulation (EC) No 1831/2003 of the European Parliament and of the Council of 22 September 2003 on additives for use in animal nutrition.

In an embodiment the composition is in a form of a liquid composition or a solid composition such as solution, dispersion, paste, pellet, powder, granule, coated granule, tablet, cake, crystal, crystal slurry, gel, extrude, precipitate, premix, or a combination thereof.

The term "promoter" refers to a portion of a gene containing DNA sequence(s) that provide for the binding of RNA polymerase and initiation of transcription. Promoter sequences are commonly, but not always, found in the 5' non-coding regions of genes.

The term "propeptide" or "pro-peptide" is a part of a protein that is cleaved during maturation or activation. Once cleaved, a propeptide generally has no independent biological function.

As used herein, the terms "domain" and "region" can be used interchangeably with the term "module".

The following abbreviations are used for amino acids:

| | | |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| X | Xaa | Any one of the above amino acids |

Substitutions are described herein by using of the following nomenclature: amino acid residue in the protein scaffold i.e. the parent sequence; position; substituted amino acid residue(s). According to this nomenclature the substitution of, for instance, a single residue of proline to lysine residue at position 121 is indicated as Pro121Lys or P121K. A substitution of any amino acid in position 121 to lysine is indicated as Xaa121Lys or X121K or 121K. As a further example, a substitution of a tyrosine in position 179 to phenylalanine, tryptophan, or leucine is indicated herein as Y179F/W/L.

As used herein, the term "expression" includes any step or all steps involved in the production of a polypeptide in a host cell including, but not limited to, transcription, translation, post-translational modification, and secretion. Expression may be followed by harvesting, i.e., recovering, the host cells or the expressed product.

In an embodiment the phytase variant has phytase activity, and an amino acid sequence with at least 80 %, at least 85 %, at least 90 %, at least 92%, at least 94%, at least 95 %, at least 96%, at least 97%, at least 98%, or at least 99% sequence identity with amino acids of SEQ ID NO: 1. In an embodiment the variant polypeptide does not have 100% sequence identity with amino acids of SEQ ID NO: 1. In an embodiment, the amino acid numbering of the variant polypeptide corresponds to that of SEQ ID NO: 1. In an alternative embodiment, the amino acid numbering of the variant polypeptide corresponds to that of SEQ ID NO: 1 partially.

In an embodiment the total number of the amino acid substitutions in the variant polypeptide, compared to the SEQ ID NO: 1, is at least two. The number of substitutions may also be at least three or at least four. The amino acid substitutions may be selected from the positions disclosed herein. In another embodiment the total number of substitutions is at least 5, at least 10, at least 15, at least 20 or at least 25; or 5, 10, 15, 20, or 25. Preferably the number of substitutions does not exceed 25, 20, 15, or 10.

Further examples of a suitable number of substitutions are 2-25 substitutions, 2-20 substitutions, 2-15 substitutions, 2-10 substitutions, and 2-5 substitutions. Further examples of a suitable number of substitutions are 3-25 substitutions, 3-20 substitutions, 3-15 substitutions, 3-10 substitutions, and 3-5 substitutions. Further examples of a suitable number of substitutions are 4-25 substitutions, 4-20 substitutions, 4-15 substitutions, 4-10 substitutions, and 4-5 substitutions.

In an embodiment the substitution or the substitutions are made in a non-conservative region of the amino acid sequence. In another embodiment the variant polypeptide comprises the substitutions specified in any claim, and additional substitutions in non-conserved region of the amino acid-sequence. The effect these additional substitutions have on the properties can be analyzed as described in the Examples.

In an embodiment the variant polypeptide, or the functional fragment, has a predicted molecular weight between 40 and 60 kDa, preferably between 43-55 kDa. The predicted molecular weight can be determined by calculating the sum of the molecular weights of the individual amino acids in the variant polypeptide, or in its functional fragment. When the predicted molecular weight of the variant polypeptide is within the above range, the structure of the variant polypeptide is similar with the parent sequence to which the substitutions are made. A predicted molecular weight in the above range may be preferable to provide a variant polypeptide which possesses similar structural or functional properties as a parent polypeptide.

In an embodiment the enzyme composition or a product containing the variant polypeptide is provided in the form of a liquid composition or a solid composition, such as solution, dispersion, paste, powder, granule, granulate, coated granulate, tablet, cake, crystal, crystal slurry, gel, or pellet. Using the present variant polypeptide in such formulations is advantageous because of the good thermostability of the variant polypeptide.

In an embodiment the variant polypeptide degrades phytic acid in a plant-based material or partially plant based material which contains phytic acid.

In an embodiment the present phytase variant is used or provided in an animal feed, and the animal is a ruminant or a non-ruminant. In another embodiment the animal is a cattle, such as beef, cow, a sheep, or a goat. In another embodiment the animal is non-ruminant such as poultry (such as broiler, layer and turkey and duck); pigs (such as piglets, growing pigs and sows); fish (such as salmonids, carp, tilapia and catfish) and crustaceans. In an embodiment the feed is animal feed intended to be fed to animals, such as any compound feed or mixture. In another embodiment feed comprises or consists of grains such as maize, wheat, oats, barley, sorghum and rice; protein sources such as soybean meal, sunflower meal and canola meal; and one or more mineral. The feed, wherein the present variant is used, has improved nutritional value compared to a feed without the variant. The present composition and the present phytase variant degrade phytic acid of the feed and thereby increase its nutritional value for the animals. The animal feed, wherein the present phytase variant or the present composition is used, can be formulated in the form of a wet composition or a dry composition.

The use of the terms "a" and "an" and "the" and similar in the context of describing the elements or features are to be construed to cover both the singular and the plural unless otherwise indicated or clearly contradicted by context. Ranges of values, including substitution sites, are merely intended to serve as a way of referring individually to each separate value falling within the range, unless otherwise indicated herein, and each separate value is thus disclosed in the specification as if it were individually recited. All methods described herein can be performed in any suitable order unless otherwise indicated herein or otherwise clearly contradicted by context. The use of any and all examples, or exemplary language (e.g., "such as", "for example", and "optionally") provided herein, is intended merely to better illustrate the embodiments without limiting the scope of the claims, unless otherwise stated. No language in the specification should be construed as indicating any non-claimed element as being an essential feature.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in this specification and attached claims are approximations that may vary depending upon the desired properties sought to be obtained by embodiments of the present disclosure. As used herein, "about" may be understood by persons of ordinary skill in the art and can vary to some extent depending upon the context in which it is used. If there are uses of the term which are not clear to a person of ordinary skill in the art, given the context in which it is used, "about" may mean up to plus 10% or up to minus 10% of the particular term.

The terms "%", "% by weight", "weight %" and "wt%" are all intended to mean unless otherwise stated, percent by weight based upon a total weight of 100% end composition weight. Thus 10% by weight means that the component constitutes 10 wt. parts out of every 100 wt. parts of total composition.

As used herein, the term "comprising" includes the broader meanings of "including", "containing", and "comprehending", as well as the narrower expressions "consisting of" and "consisting only of".

In an embodiment the process steps are carried out in the sequence identified in any aspect, embodiment, or claim. In another embodiment any process step specified to be carried out to a product or an intermediate obtained in a preceding process step is carried out directly to said product or intermediate, i.e. without additional, optional or auxiliary processing steps that may chemically and/or physically alter the product or intermediate between said two consecutive steps.

In an embodiment the present process is an industrial process. In another embodiment the industrial process excludes small scale methods such as laboratory scale methods that are not scaled up to volumes used in industry.

The appended claims define the scope of protection. Any example and technical description of an apparatus, product and/or method in the description and/or drawing not covered by the claims is presented not as an embodiment of the invention but as background art or example useful for understanding the invention.

### EXAMPLES

The following examples are provided to better illustrate the claimed invention. The examples are not to be interpreted as limiting the scope of the invention, which is determined by the claims. To the extent that specific materials are mentioned, it is merely for purposes of illustration and not intended to limit the invention. One skilled in the art may develop equivalent means or reactants without exercising inventive capacity and without departing from the scope of the invention. It shall be understood that many variations can be made in the procedures described herein while remaining within the scope of the claims.

### Example 1. Design of variants with improved thermostability and relative IP4 activity

Computational analysis and design basing on phytase structure was performed to improve the phytate degrading activity and thermostability of SEQ ID NO:1 phytase.

Molecular structures of the most probable IP4 intermediates of 6-phytases according to Ariza *etal.* 2013 (doi:10.1371/journal.pone.0065062) were constructed. All atoms not part of the protein chain in the structural model of 6-phytase were removed, hydrogens were added according to a pH of 5.0, and enzyme-substrate interactions were studied using accelerated molecular dynamics approach. Based on these studies the most promising substitution candidates of increased phytate degradation activity were identified.

Molecular dynamics studies based on the crystal structure of E. *coli* 6-phytase (1DKP) were performed to reveal temperature sensitive positions.

After the above designs, variants with single mutations were produced in *Trichoderma reesei* and these variant phytases were analysed for their thermostability and IP6 and IP4 activities in a way described in the Examples 2-4 of this application. The candidates with the best properties were tested in GIT assay *(in vitro* application trial) like described in Example 6 and the best ones, basing on all the analysis performed, were tested for their efficiency in feeding trials.

Based on the laboratory experiments, *in vitro* and *in vivo* application trial results, and additional computation designs, variants were designed in which the beneficial mutations were combined. These designed phytase variants, aimed to have both improved phytate degrading ability and thermostability compared to SEQ ID NO:1 are described in detail in Table 1. The variants of Table 1 have substitutions in positions that improve thermal stability of the enzyme and allow binding of IP substrates, especially IP4 substrates, to the active site, thereby enhancing phytate degradation activity.

Further, based on the computational design and/or characterisation results each of the positions listed for each variant in Table 1 is relevant for the binding of IP4 substrate to the substrate binding site, or to improving thermal stability of the enzyme. Additionally, the said positions can be used either alone or in any combination with one or more of the substitution positions listed in Table 1 in order to improve IP4 degradation and/or stability of the enzyme, as evidenced by the computational and other experiments. Preferable combinations of the identified substitution sites were analysed in further studies by preparing variants having the variant codes shown in Table 1.

**Table 1. List of phytase variants designed for production and characterisation. The amino acid numbering corresponds to the amino acid numbering of the mature parent phytase molecule presented in SEQ ID NO: 1.**

| **Variant code** | **Mutation(s)** |
|---|---|
| BB70 | Q30R, S80P, R94L, T118L, P121K, N126H, N176P, L179K, K180N, K183A, D204N, W211V, S212G, Q224E, Q227E, V253Y, K276M, T277A, Q287S, E315G, L352M, A380P |
| BB71 | Q30R, S80P, R94L, T118L, P121K, N126H, N176P, L179K, K180N, K183A, D204N, W211V, S212G, M216T, Q224E, Q227E, V253Y, K276M, T277A, Q287S, E315G, L352M, A380P |
| BB72 | Q30R, S80P, R94L, T118L, P121K, N126H, N176P, L179K, K180N, D204N, W211V, S212G, M216T, Q224E, Q227E, V253Y, K276M, T277A, Q287S, E315G, L352M, A380P |
| BB73 | Q30R, S80P, R94L, T118L, N126H, N176P, L179K, K180N, K183A, D204N, W211V, S212G, M216T, Q224E, Q227E, V253Y, K276M, T277A, Q287S, E315G, L352M, A380P |
| BB74 | Q30R, S80P, R94L, T118L, P121K, N126H, N176P, L179K, K180N, K183A, D204N, W211V, S212G, Q224E, Q227E, V253Y, Q258N, K276M, T277A, Q287S, E315G, L352M, A380P |
| BB75 | Q30R, S80P, R94L, T118L, N126H, N176P, L179K, K180N, K183A, D204N, W211V, S212G, M216T, Q224E, Q227E, V253Y, Q258N, K276M, T277A, Q287S, E315G, L352M, A380P |
| BB76 | Q30R, S80P, R94L, T118L, P121K, N126H, N176P, L179K, K180N, K183A, D204N, W211V, S212G, M216T, Q224E, Q227E, V253Y, Q258N, K276M, T277A, Q287S, E315G, L352M, A380P |
| BB78 | Q30R, A56S, V67I, S80P, R94L, D107N, T118L, P121K,N126H, D154N, Q174E, N176P, L179K, K180N, A202S, D204N, W211V, S212G, Q224E, Q227E, V253Y, L271I, K276M, T277A, Q285E, Q287S, G302A, E315G, N344D, L352M, A380P, G395A |
| BB79 | Q30R, A56S, V67I, S80P, R94L, D107N, T118L, P121K,N126H, D154N, Q174E, N176P, L179K, K180N, A202S, D204T, W211V, S212G, Q224E, Q227E, V253Y, L271I, K276M, T277A, Q285E, Q287S, G302A, E315G, N344D, L352M, A380P, G395A |
| BB80 | Q30R, A56S, V67I, S80P, R94L, D107N, T118L, P121K, N126H, D154N, Q174E, N176P, L179K, K180N, A202S, D204N, W211V, S212G, M216T, Q224E, Q227E, V253Y, L271I, K276M, T277A, Q285E, Q287S, G302A, E315G, N344D, L352M, A380P, G395A |
| BB81 | Q30R, A56S, V67I, S80P, R94L, D107N, T118L, N126H, D154N, Q174E, N176P, L179K, K180N, A202S, D204N, W211V, S212G, M216T, Q224E, Q227E, V253Y, L271I, K276M, T277A, Q285E, Q287S, G302A, E315G, N344D, L352M, A380P, G395A |
| BB82 | Q30R, A56S, V67I, S80P, R94L, D107N, T118L, P121K, N126H, D154N, Q174E, N176P, L179K, K180N, A202S, D204T, W211V, S212G, M216T, Q224E, Q227E, V253Y, L271I, K276M, T277A, Q285E, Q287S, G302A, E315G, N344D, L352M, A380P, G395A |
| BB83 | Q30R, A56S, V67I, S80P, R94L, D107N, T118L, P121K, N126H, D154N, Q174E, N176P, L179K, K180N, K183A, A202S, D204N, W211V, S212G, M216T, Q224E, Q227E, V253Y, L271I, K276M, T277A, Q285E, Q287S, G302A, E315G, N344D, L352M, A380P, G395A |
| BB84 | Q30R, A56S, V67I, S80P, R94L, D107N, T118L, P121K, N126H, D154N, Q174E, N176P, L179K, K180N, K183A, A202S, D204N, W211V, S212G, M216T, Q224E, Q227E, V253Y, L271I, K276M, Q285E, Q287S, G302A, E315G, N344D, L352M, A380P, G395A |
| BB85 | Q30R, A56S, V67I, S80P, R94L, D107N, T118L, P121K, N126H, D154N, Q174E, N176P, L179K, K180N, A202S, D204N, W211V, S212G, M216T, Q224E, Q227E, V253Y, L271I, K276M, Q285E, Q287S, G302A, E315G, N344D, L352M, A380P, G395A |

### Example 2. Production of phytase variants

Standard molecular biology methods were used in the isolation and enzyme treatments of DNA (e.g., isolation of plasmid DNA, digestion of DNA to produce DNA fragments), in *E. coli* transformations, sequencing etc. The basic laboratory methods used were either as described by the enzyme, reagent or kit manufacturer or as described in the standard molecular biology handbooks or as described in the following examples.

The phytase genes encoding the designed phytase variants (Example 1, Table 1) were ordered as synthetic genes using codons optimised for expression in *Trichoderma reesei.*

The phytases in the constructions were expressed from *T. reesei cbh1* (*cel7A)* promoter using a carrier polypeptide (CBM and linker) encoding sequence from *T. reesei cbh2 (cel6A).* A Kex2 protease cleavage site was included between the carrier polypeptide and phytase like described in Paloheimo *et al.* 2003. The transcription was terminated using *cbh2* terminator, followed in the construction by a synthetic amdS marker gene. In addition, the constructions contain *cbh1* 3' and 5' flanking regions for optionally targeting the expression vector into the *cbh1* locus (Figure 1).

Circular expression plasmids were transformed in T. reesei protoplasts. The transformants were selected on plates containing acetamide as the sole nitrogen source. The host strain used lacks the four major endogenous T. *reesei* cellulases: CBHI/Cel7A, CBHII/Cel6A, EGI/Cel7B and EGII/Cel5A. The transformations were performed according to Penttilä et al., 1987, with the modifications described in Karhunen et al., 1993. Alternatively, CRISPR-Cas technology can be used in transformations.

The transformants were sporulated on potato dextrose agar (PDA) prior to cultivation.

The transformants were cultivated on 96-well plates (Havukainen et al., 2020) to analyse the phytase production of the transformants. Phytase activity of the recombinant variant phytases was measured from the culture supernatants as release of inorganic phosphate from sodium phytate as described in Example 3. Production of the recombinant protein was also detected from the culture supernatant by sodium dodecyl sulphate polyacrylamide gel electrophoresis (SDS-PAGE).

All the transformants produced phytase activity. Transformants producing variant phytases with the highest phytase activity and the reference strain producing the parent phytase were purified on selection plates through single conidia prior to sporulating them on PDA. The purified, selected transformants were cultivated in shake flasks and/or bioreactors in complex cellulase-inducing medium to obtain material for further characterisation and application tests.

### Example 3. Phytase activity assays

Phytase activity was analysed from culture supernatants using IP6 and isolated IP4 form as a substrate as described below.

The phytic acid dodecasodium salt (IP6) used in the analysis was purchased from LabChem (C₆H₆Na₁₂O₂₄P₆, EE05501). Method to generate mainly IP4 specific isomer fraction was performed in 4 steps. In a first step Quantum Blue phytase was immobilized on 5ml HiTrap NHS-activated sepharose column from General Electric (Boston, USA) as described in Greiner & Konietzky, (1996). In a second step, the immobilized phytase degraded IP6 in 0.1 M Na-acetate buffer pH 5.0 stepwise to lower inositol phosphates. The flowrate of 5ml/min achieved the highest portion of IP4. The next step was used to remove phosphate from the solution and separate IP4 from other undesired inositol phosphates. Therefore, a manually packed anion exchange column with AG1-x4 resin from Bio-Rad (Hercules, USA) was loaded with the produced inositol phosphate mix and IP₄ was eluted with 0.5 M HCl. In the last step, HCl was removed with a rotating evaporator and IP4 re-dissolved in water.

The activity of samples from the microtiter plate cultivations for transformant selection (Example 2) was screened using Fluent^{®} automation workstation (Tecan Group Ltd, Männedorf, Switzerland) as follows. The samples used in the assay are diluted in a 0.2 M citrate buffer (pH 5.0) containing 0.01 % Tween 20 (Merck 822184). 0.01 % of Tween 20 is added also to the substrate solution. The substrate concentration used in this analysis is 12.7 mM. 200 µl of sample dilution and 200 µl of substrate are mixed and incubated at 37°C. After exactly 15 min incubation 400 µl of 15 % (w/v) TCA solution (Trichloroacetic acid, CCl₃COOH, Merck 807) is added to the mixture to stop the reaction. 25 µl of reaction mixture is transferred into another well and 225 µl of water is added to make 1:10 dilution. 250 µl of colour reagent consisting of three volumes of 1 M sulphuric acid (H₂SO₄, Merck 731), one volume of 2.5 % (w/v) ammonium molybdate ((NH₄)₆Mo₇O₂₄ · 4 H₂O, Merck 1182) and one volume of 10 % (w/v) ascorbic acid (C₆H₈O₆, AnalaR Normapur 20150) is added and the colour reaction is incubated for 20 min at 50 °C. After the incubation the absorption is measured at 820 nm. The absorbance of the sample is compared to that of a reference sample which is the SEQ ID NO: 1.

The activity from the shake flask and fermentation cultivation samples (Example 2) was analysed using a phytase activity assay (PPU). In PPU analysis one activity unit is the quantity of enzyme that liberates 1 µmol of inorganic phosphate per one minute from sodium phytate at pH 4.0 and at 37 °C in a 15 min reaction time. The substrate concentration used in this analysis for both IP6 and IP4 form is 12.7 mM.

The samples used in the assay are diluted in a reaction buffer (0.2 M citrate buffer, pH 4.0) and 1 ml of enzyme solution is used in the analysis. 1 ml of substrate is added to the enzyme solution and after incubating the mixture at 37°C for exactly 15 min, the reaction is stopped by adding 2 ml of 15 % (w/v) TCA solution (Trichloroacetic acid, , Merck 807). The reaction mixture is cooled to room temperature and after this 1:10 dilution is done by mixing 0.2 ml of the mixture and 1.8 ml of water in a test tube. 2.0 ml of freshly made colour reagent is added to the tube and mixed. The colour reagent consists of three volumes of 1 M sulphuric acid (H₂SO₄, Merck 731), one volume of 2.5 % (w/v) ammonium molybdate ((NH₄)₆MO₇O₂₄ ·4 H₂O, Merck 1182) and one volume of 10 % (w/v) ascorbic acid (C₆H₈O₆, AnalaR Normapur 20150). The tubes are incubated at 50 °C for 20 min and cooled to room temperature. After this the absorption is measured at 820 nm against the enzyme blank. For the enzyme blank the substrate is added after the TCA and the 15 min incubation is passed. The amount of liberated phosphate is determined via a standard curve of the color reaction with a phosphate solution of known concentration.

The activity for the samples used in gastrointestinal tract test (GIT) (Example 6) was analysed by an internal validated phytase method (FTU assay). In FTU assay inorganic phosphate released from sodium phytate substrate by the hydrolytic enzymatic action of phytase is detected. Colour formation, which is measured spectrophotometrically, is the result of molybdate and vanadate ions complexing with inorganic phosphate. One phytase unit (FTU) is the quantity of enzyme that liberates 1 µmol of inorganic phosphate per minute from sodium phytate at 37 °C, pH 5.50, using 60 min incubation time.

In the assay, 2.0 ml of 1 % sodium phytate substrate (LabChem EE05501, in 250 mM sodium acetate buffer, pH 5.5 and including 1 mM CaCl₂·2H₂O and 0.01% Tween 20) is pipetted to plastic centrifuge tubes. The substrate tubes are pre-incubated for 5-10 minutes at 37 °C after which 1.0 ml of diluted enzyme sample is added. After exactly 60 min incubation 2.0 ml of colour stop solution is added and tube contents are mixed by vortexing. Enzyme blanks are prepared like the sample but the colour stop solution is added to the substrate tubes prior to addition of the diluted enzyme sample. For colour reaction the tubes are incubated for 20 min at room temperature after which they are centrifuged at 4000 rpm for 10 minutes. The sample absorbance is measured against an enzyme blank at 415 nm. For the activity units, a potassium phosphate standard curve (pH 5.50) is prepared (dried KH₂PO₄, Merck 1.04873.1 is used for the standard; drying at 105 °C for 2 hours before weighting).

The stop solution is prepared as follows (preparation just prior to use): for 100 ml of colour stop solution, 25 ml of stock ammonium heptamolybdate (20 g of (NH₄)₆MO₇O₂₄ ·4H₂O, Merck 1182 in 180 ml of water, add 2 ml of ammonium hydroxide (NH₄OH, Sigma-Aldrich 221228 28-30 %), final volume 200 ml) is mixed with 25 ml of stock ammonium vanadate solution (0.47 g of ammonium vanadate (NH₄VO₃, Riedel de Haen 31153) in 160 ml of water; once the completely dissolved, 4 ml of 22.75 % nitric acid solution is added, final volume 200 ml). Then, 16.5 ml of 22.75 % nitric acid solution (HNO₃, Merck 1.00456) is added after which distilled water is added to make up the volume to 100 ml in volumetric flask.

**Table 2. The ratio of IP4 activity to IP6 activity measured from the shake-flask cultivation supernatants of selected variants. The numbers presented here are relative activities compared to SEQ ID NO: 1. The results show that the variants have an increased ratio of IP4 to IP6 activity compared to SEQ ID NO: 1.**

| Variant code | IP4/IP6 |
|---|---|
| SEQ ID NO: 1 | 1.00 |
| BB70 | 1.13 |
| BB71 | 1.56 |
| BB72 | 1.41 |
| BB73 | 1.29 |
| BB74 | 1.10 |
| BB75 | 1.16 |
| BB76 | 1.26 |
| BB78 | 1.32 |
| BB79 | 1.52 |
| BB80 | 1.43 |
| BB81 | 1.21 |
| BB82 | 1.52 |
| BB83 | 1.47 |
| BB84 | 1.49 |
| BB85 | 1.39 |

### Example 4. Analysis of unfolding temperatures of phytases

The unfolding temperatures of phytase variants (Examples 1 and 2) were analysed from culture supernatants using Prometheus NT.48 equipment (NanoTemper GmbH, Munich, Germany).

Prior to the measurements the samples were centrifuged for 10 min at 16000g at 4°C to remove any large aggregates. Standard-grade glass capillaries (NanoTemper GmbH) were filled with 10-15 µl of the sample, excitation light was preadjusted to get adequate fluorescence signal for F330 and F350, and samples were measured in temperature range 20-110°C with temperature slope of 1°C/min.

The inflection point of the unfolding curve, also known as the melting temperature (Tₘ), is determined from the experimental derivative or curve fitting. Protein samples with higher Tₘ tend to be more stable.

The parent phytase (SEQ ID NO:1) was used as a reference in the analysis.

The unfolding temperatures of the best variants were improved up to 3.2°C. The results from Prometheus analysis of selected variants are shown in Table 3.

**Table 3. Unfolding temperatures of parent and selected phytase variants. The unfolding temperatures were measured from culture supernatants obtained from 96-well cultivations using Prometheus equipment NT.48.**

| **Variant code** | **Unfolding temperature (°C)** |
|---|---|
| SEQ ID NO: 1 | 87.9 |
| BB70 | 90.6 |
| BB71 | 90.6 |
| BB72 | 90.6 |
| BB73 | 91.1 |
| BB74 | 89.6 |
| BB75 | 90.4 |
| BB76 | 89.7 |
| BB78 | 90.3 |
| BB79 | 90.1 |
| BB80 | 90.5 |
| BB81 | 91.1 |
| BB82 | 90.9 |
| BB83 | 90.7 |
| BB84 | 90.0 |
| BB85 | 90.8 |

### Example 5. Stability test in feed processing

For stability tests in feed processing the phytase samples were added to a wheat-soya bean meal based diet which was conditioned for 30 seconds at temperatures of 90°C and 95°C followed by pelleting through a 3 mm die. The pellets were cooled and analysed for phytase activity.

The feed contained approximately 65% wheat, 28% soybean meal, 4% soya oil, 1% monocalcium phosphate, 1.4% limestone, 0.4% salt and a premix (0.5%) of vitamins and trace minerals. Part of that feed was used to produce a premix with the phytase, that was added to the total feed amount and mixed in a horizontal mixer for 10 minutes. After taking the mash sample the feed was heated to the target temperatures by adjusting steam addition when passing the cascade mixer before running though the pellet die. For each temperature, a sample was taken 10 minutes after the target conditioning temperature was achieved (as measured in the feed just prior to pelleting).

Samples of pelleted feeds were taken and cooled by cold air stream. On a sample splitter feeds were homogeneously sub-sampled for analysis using the phytase in feed assay method (ISO 30024:2009; Animal feeding stuffs - Determination of phytase activity).

Phytase recovery was calculated relative to the activity in the corresponding feed mash sample pre-processing.

As an example, the phytase recovery compared to the corresponding activity in the mash feed is shown for BB81 in Fig. 3. The recovery of BB81 after feed processing at 90°C and 95°C was clearly higher as analysed for phytase SEQ ID NO:1. This demonstrates that the improved unfolding temperatures of the variants (Table 3) translate into high stability under conditions common in commercial feed pelleting. A high unfolding temperature of a variant thus indicates stability in feed processing involving high temperature.

### Example 6. Gastrointestinal tract test (GIT) results

The comparison of selected novel phytase candidates in their ability to degrade phytate in feed materials was done using an in vitro gastrointestinal simulation test system (GIT). SEQ ID NO:1 was used as a reference.

Phytases to be tested are added with a defined activity level (500 FTU/kg) to a corn soyabean meal based broiler feed following a three-step continuous in vitro test simulating the animal digestive conditions. The reactions were run at 40°C and at corresponding pHs and changes of pH as in the crop, gizzard and small intestine of broilers. Additionally, digestive proteases are added. To succeed in the GIT assay, the phytase needs to have a combination of beneficial biochemical properties. It needs to resist and act at different pHs at the temperature of the digestive tract while being resistant to proteases occurring in animals digestive tract. Details of the in vitro test system are described by Sommerfeld et al., 2017.

At the end of the in vitro test inositol phosphates are extracted and phytase removed from the supernatant before analysis of inositol phosphates (IP6 - IP4) using high performance liquid chromatography method (HPLC) according to Blaabjerg et al. 2010.

The results obtained from the GIT test are shown in Figure 2. Residual amounts of IP6, IP5 and IP4 analysed at the end of the GIT simulation were calculated together and compared to results obtained without any phytase used (Blank) or the same amount of SEQ ID NO: 1 phytase used.

Data demonstrates that the variants degraded phytate significantly better compared to SEQ ID NO: 1 phytase. The remaining amount of IP6, IP5 and IP4 calculated together was 2% (BB71); 11% (BB73); 15% (BB75); 12% (BB76); 9% (BB78); 4% (BB79); 5% (BB80); 16% (BB81) and 62% (BB86) compared to the amount analysed after the *in vitro* treatment of phytate with SEQ ID NO: 1 phytase.

### Example 7. Feeding trials

The phytase variants described above can be used in animal feeding, alone or in combination with other enzymes, to release phosphorus from *myo*-inositol 1, 2, 3, 4, 5, 6 hexa-kis phosphate (InP6). InsP6 degradation in the animals intestinal tract improves phosphorus digestibility. Additionally, antinutritional properties due to chelates with proteins, minerals and trace minerals of InsP6 and its lower inositol phosphates are reduced by phytases.

The efficiency in animal feeding of some of the recombinant phytase variants of the invention was confirmed in growth performance studies with broilers. Ultrafiltrate of the fermentation broth including the recombinant phytase was dried and defined activity levels were applied to the trial diets and the performance of the birds was compared to broilers fed the same diet composition without phytase or with phytase of SEQ ID NO:1.

**Broiler trial 1:** Day old male Cobb 430 broilers were distributed to the different treatments (17 pens x 26 birds each). Phosphorus (P) and calcium (Ca) content of the corn-soybean meal based diet was reduced compared to the breeders recommendation in the diets (available P 1.5 /kg and Ca 8.0 g/kg). Phytase SEQ ID NO: 1 or variants BB73, BB78 or BB81 were added with a target activity of 500 FTU/kg feed. At day 21 birds fed the diet added with BB73, BB78 or BB81 achieved higher body weight gain, than birds fed with the same diet added with SEQ ID NO: 1 phytase. Feed efficiency was either the same as in SEQ ID NO: 1 fed birds or improved.

**Broiler trial 2:** Day old male Ross 308 broilers were distributed to the different treatments (8 pens x 12 birds each). Phosphorus and calcium content of the corn-soybean-rapeseed meal basal diet was reduced (BD: available P content 1.9 g/kg and Ca 7.5 g/kg, respectively). Phytase SEQ ID NO: 1 or variants BB73, BB78 and BB81 were added with a target activity of 500 FTU/kg feed. At day 21 birds fed the diet added with SEQ ID NO:1; BB73, BB78 or BB81 achieved a 13%, 15%, 16% and 13% higher body weight gain, respectively, than birds fed the unsupplemented basal diet. Feed conversion ratio was either the same as in SEQ ID NO: 1 fed birds or improved.

The results show that the designed variants are efficient in animal feeding.

### REFERENCES

Ariza A, Moroz OV, Blagova EV, Turkenburg JP, Waterman J, Roberts SM, Vind J, Sjøholm C, Lassen SF, De Maria L, Glitsoe V, Skov LK and KS Wilson. 2013. Degradation of phytate by the 6-phytase from Hafnia alvei: a combined structural and solution study. PloS one 2013. 8(5), e65062. https://doi.org/10.1371/journal.pone.0065062

Bedford M.R. and C.L. Walk. 2016. Reduction of phytate to tetrakisphosphate (IP4) to trisphosphate (IP3), or perhaps even lower, does not remove its antinutritive properties. In: Phytate destruction - consequences for precision animal nutrition. Eds. Walk, C.L., Kühn, I., Stein, H.H., Kidd, M.T. and Rodehutscord, M. Wageningen 20 Academic publishers: 45-52. Blaabjerg, K., H. Jørgensen, A. H. Tauson, and H. D. Poulsen. 2010. Heat-treatment, phytase and fermented liquid feeding affect the presence of inositol phosphates in ileal digesta and phosphorus digestibility in pigs fed a wheat and barley diet. Journal Article 4:876-885. Greiner R. and U. Konietzny. 1996. Construction of a bioreactor to produce special breakdown products of phytate. Journal of Biotechnology, 48 (1-2): 153-159.

Havukainen S, Valkonen M, Koivuranta K and Landowski CP. 2020. Studies on sugar transporter CRT1 reveal new characteristics that are critical for cellulase induction in Trichoderma reesei. Biotechnol. Biofuels 2020 Sep 14;13:158. doi: 10.1186/s13068-020-01797-7. eCollection 2020.

Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.

Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.

Penttilä M, H Nevalainen, M Rättö, E Salminen and J Knowles. 1987. A versatile transformation system for the cellulolytic filamentous fungus Trichoderma reesei. Gene 61:155-164.

Sievers F, A Wilm, D Dineen, TJ Gibson, K Karplus, WLi, R Lopez, H McWilliam, M Remmert, J Söding, JD Thompson and DG Higgins 2011. Fast, scalable generation of high-quality protein multiple sequence alignments using Clustal Omega. Mol. Syst. Biol. 7:539.

Sommerfeld M, Schollenberger, L Hemberle and M Rodehutscord 2017 Modification and application of an in vitro assay to examine inositol phosphate degradation in the digestive tract of poultry. Science of Food and Agiculture; 97 (12), p 4219-4226; doi.org/10.1002/jsfa.8297 Zeller E, M Schollenberger, I Kühn and M Rodehutscord. 2015. Hydrolysis of phytate and formation of inositol phosphate isomers without or with supplemented 30 phytases in different segments of the digestive tract of broilers. Journal Nutritional Science 4, e1:1-12.

The foregoing description has provided by way of non-limiting examples of particular implementations and embodiments a full and informative description of the best mode presently contemplated by the inventors for carrying out the invention.

It is however clear to a person skilled in the art that the invention is not restricted to details of the embodiments presented in the foregoing, but that it can be implemented in other embodiments using equivalent means or in different combinations of embodiments without deviating from the characteristics of the invention.

## Claims

1. A variant polypeptide comprising an amino acid sequence having at least 80% amino acid sequence identity with SEQ ID NO: 1 and comprising amino acid substitutions at:
the position 126; and
at least one position selected from 30, 80, 94, 118, 176, 179, 212, 224, 227, 253, 287, 315, and 380; and
wherein the variant polypeptide has phytase activity; and
wherein the amino acid numbering corresponds to the amino acid numbering of the SEQ ID NO: 1.

2. The variant polypeptide of claim 1 having a histidine at the position 126.

3. The variant of claim 1 or 2 further comprising a substitution at the position 121 and/or 216.

4. The variant polypeptide of claim 3, wherein the substitution at the position 121 and/or 216 is selected from 121K and 216T, preferably selected from P121K and M216T.

5. The variant polypeptide of any one of claims 1-4 having an increased IP4 degradation activity compared to a phytase having the amino acid sequence SEQ ID NO: 1.

6. The variant polypeptide of any one of claims 1-5 comprising at least one further amino acid substitution at a position selected from 56, 67, 107, 154, 174, 180, 183, 202, 204, 211, 258, 271, 276, 277, 285, 302, 344, 352, and 395.

7. The variant polypeptide of claim 6, wherein the at least one further amino acid substitution results into the presence of at least one of the following amino acids: 56S, 67I, 107N, 154N, 174E, 180N, 183A, 202S, 204N, 204T, 211V, 258N, 2711, 276M, 277A, 285E, 302A, 344D, 352M, or 395A.

8. The variant polypeptide of any one of claims 6-7, wherein the at least one further amino acid substitution is a substitution selected from A56S, V67I, D107N, D154N, Q174E, K180N, K183A, A202S, D204N, D204T, W211V, Q258N, L271I, K276M, T277A, Q285E, G302A, N344D, L352M, or G395A.

9. The variant polypeptide of any one of clams 1-8 having an unfolding temperature of at least 88°C, preferably at least 89°C, and even more preferably at least 90°C.

10. The variant polypeptide of any one of claims 1-9 having an improved thermostability compared to a phytase having the amino acid sequence SEQ ID NO: 1.

11. The variant polypeptide of any one of claims 1-10 comprising:
a set of substitutions at the positions 30, 80, 94, 118, 121, 126, 176, 179, 180, 183, 204, 211, 212, 216, 224, 227, 253, 276, 277, 287, 315, 352, and 380; or a set of substitutions 30R, 80P, 94L, 118L, 121K, 126H, 176P, 179K, 180N, 183A, 204N, 211V, 212G, 216T, 224E, 227E, 253Y, 276M, 277A, 287S, 315G, 352M, and 380P; or
a set of substitutions Q30R, S80P, R94L, T118L, P121K, N126H, N176P, L179K, K180N, K183A, D204N, W211V, S212G, M216T, Q224E, Q227E, V253Y, K276M, T277A, Q287S, E315G, L352M, and A380P.

12. The variant polypeptide of any one of claims 1-10 comprising:
a set of substitutions at the positions 30, 80, 94, 118, 126, 176, 179, 180, 183, 204, 211, 212, 216, 224, 227, 253, 276, 277, 287, 315, 352, and 380; or a set of substitutions 30R, 80P, 94L, 118L, 126H, 176P, 179K, 180N, 183A, 204N, 211V, 212G, 216T, 224E, 227E, 253Y, 276M, 277A, 287S, 315G, 352M, and 380P; or
a set of substitutions Q30R, S80P, R94L, T118L, N126H, N176P, L179K, K180N, K183A, D204N, W211V, S212G, M216T, Q224E, Q227E, V253Y, K276M, T277A, Q287S, E315G, L352M, and A380P.

13. The variant polypeptide of any one of claims 1-10 comprising:
a set of substitutions at the positions 30, 56, 67, 80, 94, 107, 118, 121, 126, 154, 174, 176, 179, 180, 202, 204, 211, 212, 224, 227, 253, 271, 276, 277, 285, 287, 302, 315, 344, 352, 380, and 395; or
a set of substitutions 30R, 56S, 67I, 80P, 94L, 107N, 118L, 121K, 126H, 154N, 174E, 176P, 179K, 180N, 202S, 204N, 211V, 212G, 224E, 227E, 253Y, 2711, 276M, 277A, 285E, 287S, 302A, 315G, 344D, 352M, 380P, and 395A; or
a set of substitutions Q30R, A56S, V67I, S80P, R94L, D107N, T118L, P121K, N126H, D154N, Q174E, N176P, L179K, K180N, A202S, D204N, W211V, S212G, Q224E, Q227E, V253Y, L271I, K276M, T277A, Q285E, Q287S, G302A, E315G, N344D, L352M, A380P, and G395A.

14. The variant polypeptide of any one of claims 1-10 comprising:
a set of substitutions at positions 30, 56, 67, 80, 94, 107, 118, 126, 154, 174, 176, 179, 180, 202, 204, 211, 212, 216, 224, 227, 253, 271, 276, 277, 285, 287, 302, 315, 344, 352, 380, and 395; or
a set of substitutions 30R, 56S, 671, 80P, 94L, 107N, 118L, 126H, 154N, 174E, 176P, 179K, 180N, 202S, 204N, 211V, 212G, 216T, 224E, 227E, 253Y, 2711, 276M, 277A, 285E, 287S, 302A, 315G, 344D, 352M, 380P, and 395A; or
a set of substitutions Q30R, A56S, V67I, S80P, R94L, D107N, T118L, N126H, D154N, Q174E, N176P, L179K, K180N, A202S, D204N, W211V, S212G, M216T, Q224E, Q227E, V253Y, L271I, K276M, T277A, Q285E, Q287S, G302A, E315G, N344D, L352M, A380P, and G395A.

15. A recombinant host cell comprising genetic elements that allow producing at least one variant polypeptide of any one of claims 1-14, and wherein the host cell is preferably selected from the group consisting of
a. filamentous fungal cells from Division *Ascomycota,* Subdivision *Pezizomycotina;* preferably from the group consisting of members of the Class *Sordariomycetes* or *Eurotiomycetes,* Subclass *Hypocreomycetidae* or *Sordariomycetidae* or *Eurotiomycetidae,* Orders *Hypocreales* or *Sordariales* or *Eurotiales,* Families *Hypocreacea* or *Nectriacea* or *Chaetomiaceae* or *Aspergillaceae,* Genera *Trichoderma* (anamorph of *Hypocrea)* or *Fusarium* or *Acremonium* or *Humicola* or *Thermothelomyces* or *Myceliophthora* or *Aspergillus;*
more preferably from the group consisting of species *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Acremonium (Cephalosporium) chrysogenum, Humicola insolens, H. grisea, Thermothelomyces thermophilus, Myceliophthora thermophila, Aspergillus niger, A. niger* var. *awamori* and *A. oryzae;*
b. bacterial cells, preferably gram-positive Bacilli such as *B*. *subtilis, B*. *licheniformis, B*. *megaterium, B*. *amyloliquefaciens, B*. *pumilus,* gram negative bacteria such as *Escherichia coli, actinomycetales* such as *Streptomyces* sp.; and
c. yeasts, such as *Saccharomyces cerevisiae, Schizosaccharomyces pombe, Pichia pastoris, Yarrowia lipolytica;* and
more preferably the host cell is selected from filamentous fungal cells such as *Trichoderma,* or from gram-positive *Bacilli* such as *Bacillus;*
most preferably from *Trichoderma reesei* or from *Bacillus subtilis* or *B*. *pumilus* or *B*. *licheniformis* or *B*. *amyloliquefaciens.*

16. A recombinant host cell comprising genetic elements configured to produce at least one variant polypeptide of any one of claims 1-14, and wherein the host cell is a transgenic plant cell.

17. An enzyme composition comprising the variant polypeptide of any one of claims 1-14 and optionally a preservative and/or a carrier.

18. A use of the variant polypeptide of any one of claims 1-14 or the enzyme composition of claim 17 in the manufacturing of feedstuff or foodstuff, feed additive, a dietary supplement, or a pharmaceutical, comprising mixing the variant polypeptide or the enzyme composition with the feedstuff, foodstuff, feed additive, dietary supplement, or pharmaceutical.

19. A method of manufacturing the variant polypeptide of any one of claims 1-14 comprising:
a. providing a polynucleotide comprising genetic elements arranged to produce the variant polypeptide of claims 1-14; and
b. expressing the polynucleotide in a recombinant host cell, preferably in the recombinant host cell of claim 15 or 16.

20. An animal feed comprising the variant polypeptide of any one of claims 1-14 or the enzyme composition of claim 17, and at least one protein source of plant origin, and
a. optionally at least one further enzyme selected from protease, amylase, phytase, xylanase, endoglucanase, beta-glucanase, mannanase, cellulase, or a combination thereof; and
b. optionally at least one carrier or ingredient selected from maltodextrin, flour, salt, sodium chloride, sulphate, sodium sulphate, or a combination thereof.

21. A feed supplement comprising the variant polypeptide of any one of claims 1-14 or the enzyme composition of claim 17; and
a. optionally at least one further enzyme selected from protease, amylase, phytase, xylanase, endoglucanase, beta-glucanase, mannanase, cellulase, or a combination thereof; and
b. optionally at least one carrier or ingredient selected from maltodextrin, flour, salt, sodium chloride, sulphate, sodium sulphate, minerals, amino acids, prebiotics, probiotics, vitamins, or a combination thereof.

22. A method of degrading or modifying material containing phytic acid or phytate, comprising treating said material with an effective amount of the variant polypeptide of any one of claims 1-14 or the enzyme composition of claim 17.
